# EUROPEAN PATENT APPLICATION

(11) **EP 1 903 042 A2**
(43) Date of publication of application: **26.03.2008**
(21) Application number: 07018654.9
(22) Date of filing: 22.09.2007
(51) Int. Cl.: C07D 417/12

(54) **An improved process for hydrogenation of 5-(substituted Benzylidene) 2,4- Thiazolidine Dione compounds to give corresponding ( +/- ) 5- (substituted Benzyl ) 2,4-Thiazolidine Dione**

(30) Priority: 22.09.2006 IN MU15162006
(71) Applicant: Cadila Pharmaceuticals Limited, Bhat, Ahmedabad 382 210 (IN)
(72) Inventor: Modi, Indravadan Ambalai, Bhat Ahmedabad - 382 210 (IN); Yadav, Mukesh Kumar, Bhat Ahmedabad - 382 210 (IN); Shukla, Manish Chandrakant, Dr., Bhat Ahmedabad - 382 210 (IN); Kagathara, Nirav Keshavlal, Dr., Bhat Ahmedabad - 382 210 (IN); Sondagar, Keval Rameshkumar, Bhat Ahmedabad - 382 210 (IN); Ponnaiah, Ravi, Dr., Bhat Ahmedabad - 382 210 (IN); Khamar, Bakulesh Mefatlal, Dr., Bhat Ahmedabad - 382 210 (IN)
(74) Representative: Towler, Philip Dean

(57) **Abstract**

The present provides a preparation of (+/-) -5- [substituted. phenyl methyl ] - 2,4-thiazolidinediones from 5-[ substituted benzylidene ]-2,4-thiazolidinediones by catalytic hydrogenation using precious metal catalyst, using DMF or formic acid [ or aqueous formic acid] and perchloric acid.

## Description

### FIELD OF THE INVENTION

The present invention relates to preparation of thiazolidine 2,4-diones, wherein 5-[substituted benzylidene]-2,4-thiazolidinediones are hydrogenated to give .(+/-)-5-[substituted phenyl methyl]-2,4thiazolidinediones.

### BACKGROUND OF THE INVENTION

Thiazolidine 2, 4-dione antihyperglycemic compounds represent a class of pharmaceuticals that act principally by decreasing insulin resistance in patients suffering from non-insulin-dependent diabetes. Therefore thiazolidinedione antihyperglycemic compounds are used typically as active substances in various pharmaceutical preparations for the treatment of type II diabetes and other disorders related to insulin resistance.

Rosiglitazone i.e. 5-[[4-[2-[Methyl-2-pyridinylamino)ethoxy]phenyl] methyl-2,4-thiazolidinedione, and Pioglitazone i.e. (+/-)[5-[[4-[-2-(5-Ethyl-2-pyridinyl)ethoxy]phenyl]methyl]-2,4]thiazolidinedione are examples, used for treating type It diabetes and other disorders relating to insulin resistance.

Ciglitazone i.e. (±)-5-[4-(1-Methylcyclohexylmethoxy)benzyl]-thiazolidine-2,4-Dione, is antidiabetic drugs of 2, 4-thiazolidine dione structural class.

US5002953 describes the process for preparation of compound of formula I by catalytic hydrogenation of compound of formula II using Pd/charcoal in solvents such as dioxane, DMF, 1:1 mixture of methanol and I, 4-dioxane.

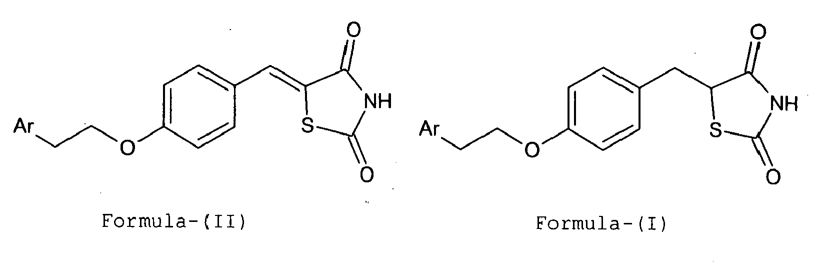

US 20020042519 discloses the reduction of 5-{4-[2-(N-methyl-N-(2-pyridyl)amino)ethoxy]benzylidene}-2,4-thiazolidinedione (IIB) using Lithium Tri-sec.butyl borohydride in THF to yield 79 % of 5-{4-[2-(N-methyl-N-(2-pyridyl)amino)ethoxy]benzyl}-2,4-thiazolidinedione (IA). In another embodiment, (IIB) is converted into (IA) in 77 % yield using lithium borohydride in a mixture of THF and pyridine and the product is isolated by aqueous quenching. In yet another embodiment of US 20020042519, (IIB) is converted to (IA) in THF using Lithium aluminum hydride in 69 % yield. The reported yield is low.

WO2005021541A2 discloses preparation of rosiglitazone free base by reduction of 5-[4-[2-(N-methyl-N-(2-pyridyl)amino)ethoxy]benzylidene]thiazolidine-2,4-dione(II), in the presence of cobalt ion , a ligand and a reducing agent , wherein the cobalt ion is provided in the form of compounds such as cobaltous chloride, cobaltous diacetate, and cobaltic chloride; the ligand is selected from the group consisting of dimethyl glyoxime, 2,2'-bipyridyl and 1,10-phenanthroline.The reducing agent is selected from group consisting of sodium borohydride ,lithium borohydride , potassium borohydride, tetraalkyl ammonium borohydride and zinc borohydride.

US20020120150 describes reduction of (Z) 5-{4-[2-(N-methyl-N-(2-pyridyl)amino)ethoxy]benzylidene}-2,4-thiazolidinedione to 5-{4-[2-(N-mefhyl-N-(2-pyridyl)amino)ethoxy]benzyl}-2,4-thiazolidinedione in 70 to 80 % yield in glacial HOAc using 10 % palladium on charcoal at 70-80 psi hydrogen pressure at about 95°C. WO 2005/049610 discloses a process for reducing an exocyclic double bond at the 5-position of 2,4-thiazolidinedione moiety of a thiazolidinedione precursor comprising the steps of : (a) preparing a solution or suspension of the thiazolidinedione precursor in a non-ether solvent medium with a base, and (b) combining the solution or suspension with a dithionite source. Preferred solvent media include aqueous N.N-dimethylformamide. Sodium dithionite is a preferred dithionite source. In particular the application discloses preparation processes for Pioglitazone, Rosiglitazone and Troglitazone.

US 6992191 describe a method of catalytically hydrogenating exocyclic double bond of a penultimate thiazolidinedione precursor comprising steps of:
a) providing a solution of the penultimate thiazolidinedione precursor in a high capacity solvent (formic acid),
b) combining the solution with a supported metal hydrogenation catalyst in a reactor, and
c) Exposing the combination of solution and hydrogenation catalyst to hydrogen gas.

The supported metal hydrogenation catalyst comprises a metal selected from the group consisting of platinum, palladium, ruthenium, rhodium, osmium, and iridium.
The invention describes use of this process for preparing Troglitazone, Pioglitazone and Rosiglitazone.

WO 2005/ 108394 A1 discloses a process for the preparation of 5-{4-[2-(N-methyl-N-(2-pyridyl)amino)ethoxy]benzyl}-2,4-thiazolidinedione (Rosiglitazone), which comprises reaction of 5-{4-[2-(N-methyl-N-(2-pyridyl)amino)ethoxy]belizylidene}-2,4-thiazolidinedione with a 1,4-dihydropyridine of general formula-III.

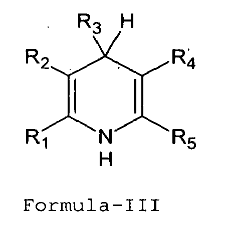

The yields reported in this process in different embodiments are 67%, 76%, 35%, 43%, 50%, 39%, and 61 %.

Bioorganic Medicinal Chemistry Letters , 1994 , vol. 4, pages 1181-84 describes the use of magnesium metal and methanol for reduction of 5-[4-[2-[N-methyl-N-(2-pyridyl)amino]ethoxy]benzylidene]thiazolidine-2,4-dione to 5-[4-[2-[N-methyl-N-(2-pyridyl)amino]ethoxy]phenyl methyl]thiazolidine-2,4-dione. The use of large quantities of magnesium metal, formation of alkoxide with methanol is inherent drawbacks of this process.

It is therefore a long felt need of the industry to provide an industrially scalable processes for reduction of 5-[substituted benzylidene]-2,4-thiazolidinediones to (+/-) -5-[substituted phenyl methyl]-2,4-thiazolidinediones.

### SUMMARY OF THE INVENTION

The present invention provides a method for preparing thiozolidine 2, 4-dione antihypergylcemics from 2,4- thiozolidinedione precursor.

The main object of the invention is to provide an improved process for the conversion of 5-[substitutedbenzylidene]-2,4 thiazolidine dione to corresponding (+/-) 5-[substituted phenyl methyl]-2,4thiazolidine diones.

Another object of the present invention is to provide an improved process for the conversion of 5-[substitutedbenzylidene]-2,4 thiazolidine diones to corresponding (+/-) 5-[substituted phenyl methyl]-2,4 thiazolidine diones using perchloric acid as hydrogenation activator in the catalytic hydrogenation process.

Another object of the invention is to provide an improved method of hydrogenating a thiazolidine 2,4-dione precursor for preparing Rosiglitazone, Pioglitazone and ciglitazone.

Yet another object of the invention is to provide an improved method for purification of rosiglitazone.

Thus in accordance with the present invention, Rosiglitazone, Pioglitazone, and ciglitazone, are prepared from their corresponding 5-substituted[benzylidene]-thiazolidine 2,4-dione precursors by catalytic hydrogenation comprising steps:
(a) Dissolving compound of foimula-II in at least one aqueous or anhydrous organic acid.
(b) Hydrogenating exocyclic double bond of arylidene compound to corresponding aryl methyl compound using perchloric acid, a precious metal catalyst and hydrogen at elevated temperature and pressure.
(c) Separating the catalyst, concentrating the filtrate, quenching in alcoholic water, followed by neutralizing and isolating a compound of formula-I.

The reduction process of the invention leads to high yields in shorter reaction time and high purity of the (+/-) 5-[substituted phenyl methyl]-2,4 thiazolidine diones.

### DETAILED DESCRIPTION

The present invention provides a method for preparing 5-(substituted phenyl methyl)2,4-thiozolidinedione antihypergylcemics from 5-(substituted arylidene)2,4-thiazolidine dione , which include the steps of hydrogenation with supported metal catalyst resulting in higher yield (of greater than 90.0%) and purity with shorter reaction times (of about 16-20 hrs).

Thus in accordance with present invention compound of formula-I

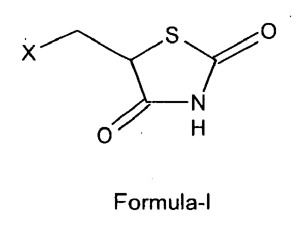

Wherein X is 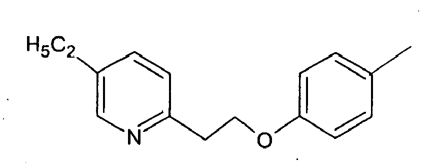 or 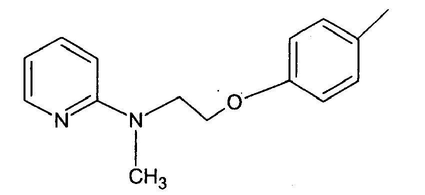 or 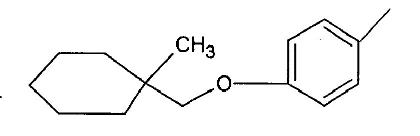 is prepared from compound of formula II

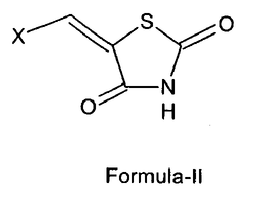

Wherein X is as defined above, in steps comprising,
(a) dissolving compound of formula-II in DMF or formic acid, adding perchloric acid,
(b) reducing exocyclic double bond of benzylidene compound to corresponding phenyl methyl compound using a precious metal catalyst and hydrogen at. elevated temperature and pressure,
(c) separating the catalyst, concentrating the filtrate , quenching in alcoholic water, followed by neutralizing and isolating compound of formula-I.

The hydrogenation catalyst is selected from the group of metals selected from platinum, palladium, ruthenium, rhodium, and iridium; preferably a palladium catalyst.

Preferred catalysts are supported metal catalyst such as palladium on carbon catalyst, typically comprising 5% to 10 % palladium on carbon. A more preferred catalyst is a 10% palladium on carbon having 50 % w/w water.

Catalyst loadings (expressed as w/w % of catalyst to substrate) in the reaction are typically in the range from 5 to 100 %, usually 10 to 80 %, preferably 50 %.

The reaction may be carried out using any suitable solvent such as DMF, formic acid, aqueous formic acid. The reaction is carried out at an elevated temperature of about 50-100°C, preferably at 60-65°C. The reaction mass is cooled after the reaction to room temperature , and the catalyst is separated by filtration, the filtrate is distilled and the residue is quenched in alcohol and water and neutralized to give compound of formula-I. The compounds of fonnula-I are isolated from the reaction by filtration and purified by conventional purification methods such as crystallization.

In still a further aspect, the present invention provides a process for preparing rosiglitazone including the step of reducing the rosiglitazone precursor comprising the above mentioned steps and isolating rosiglitazone.

In still a further aspect, the present invention provides a process for preparing pioglitazone including the step of reducing the pioglitazone precursor comprising the above mentioned steps and isolating pioglitazone.

Depending on the 2,4-thiazolidinedione precursor selected, the process as described herein may lead to the corresponding 2,4-thiazolidinedione antihyperglycemic compound which is obtained in a high yield and with high purity.

The free base of the 2,4-thiazolidinedione antihyperglycemic compound may be further purified.

In one of the preferred embodiments rosiglitazone obtained is purified by treating rosiglitazone in a mixture 4:1 mixture of Ethyl acetate: dichloromethane in presence of triethyl amine at 0-45°C , preferably at 20-35°C for about 15 min to 4 hours preferably for 1 hour. This treatment was repeated if required. The product resulted in about 85 % yield based on crude rosiglitazone having Chromatographic Purity of about 98.5 -99.3%, and having any unknown individual impurity < 1.0% and having starting benzylidene impurity <0.15 %

Purified 2,4-thiazolidinedione antihyperglycemic compound can be converted to its pharmaceutically acceptable salt, e. g. to the hydrochloride in the case of pioglitazone, or e.g. to the maleate in the case of rosiglitazone, by known conventional methods.

The preparation of 2,4-thiazolidinedione antihyperglycemic compounds, e. g. pioglitazone, rosiglitazone, ciglitazone, by reducing corresponding 2,4-thiazolidinedione precursors by using a perchloric acid as described in the present invention, involves a novel reduction process which is attractive both from economic and ecological standpoints.

The reduction process of the invention displays the use of perchloric acid, relates to novel technique for the reduction of the 2,4-thiazolidinedione precursors, which lead to high yields in shorter reaction time and higher purity of 2,4- thiazolidinedione antihyperglycemic compounds, such as pioglitazone, rosiglitazone, ciglitazone. The process of the present invention also offers, the advantages of using reaction agents which are readily commercially available, cheap, and ecologically "unrisky" and which avoid potentially dangerous handling.

Present invention is illustrated with following examples which do not limit the scope of this invention

### Example-1

A mixture of 5-{4-[2-(N-methyl-N-(2-pyridyl)amino)ethoxy]benzylidene}-2,4-thiazolidinedione (10 gm), DMF (150 ml), 10 % Palladium on charcoal [with 50 % w/w moisture] (8 gms) and 70 % w/v perchloric acid (3 ml) were charged in an autoclave. The autoclave was freed from air by pressurizing with nitrogen and degassed 3 to 4 times and finally pressurized with hydrogen till 5 kg. The reaction mass was stirred at 500 rpm and heated to 100°C and maintained at this temperature for about 12 hours to give a conversion rate of >98 %. The reaction mass was filtered on Buchner funnel and filtrate was distilled under reduced pressure. The residue was quenched with isopropyl alcohol, water and neutralized to give crude rosiglitazone.

### Example-2

A mixture of 5-{4-[2-(N-methyl-N-(2-pyridyl)amino)ethoxy]benzylidene}-2,4-thiazolidinedione (10 gm), formic acid (150 ml), 10 % Palladium on charcoal [with 50 % w/w moisture] (8 gms) and 70 % w/v perchloric acid (3 ml) were charged in an autoclave . The autoclave was freed from air by pressurizing with nitrogen and degassed 3 to 4 times and finally pressurized with hydrogen till 5 kg. The reaction mass was stirred at 500 rpm and heated to 60°C and maintained at this temperature for about 12 hrs. The reaction mass was filtered on Buchner funnel and filtrate was distilled under reduced pressure. The residue was quenched with isopropyl alcohol and water and neutralized to give crude rosiglitazone.

### Example-3

A mixture of 5-{4-[2-(N-methyl-N-(2-pyridyl)amino)ethoxy]benzylidene}-2,4-thiazolidinedione (250 gm), formic acid (2500 ml), 10 % Palladium on charcoal [with 50 % w/w moisture] (125 gms) and 70 % w/v perchloric acid (70 ml) were charged in an autoclave . The autoclave was freed from air by pressurizing with nitrogen and degassed 3 to 4 times and finally pressurized with hydrogen till 5 kg. The reaction mass was stirred at 500 rpm and heated to 60°C-65°C and maintained at this temperature till near complete conversion (less than 0.5% of starting material remains) for about 25-30 hrs. The reaction mass was cooled to 30-35°C, filtered on Buchner funnel to separate catalyst and filtrate was distilled under reduced pressure. The residue was quenched with 1:1 aqueous isopropyl alcohol, and neutralized with aqueous ammonia, precipitated product, filtered , washed with water and dried to give crude rosiglitazone
Yield= 220-225 gm
% yield= 88-90 %
% purity~ 95 %
Loss on drying not more than 2 %

### Example-4

### Preparation of pure rosiglitazone

Crude rosiglitazone [100 gm] is stirred with 500 ml mixture of 4:1, [Ethyl acetate: dichloromethane] and 4 ml of triethyl amine at 30-35°C for about 60 minutes, filtered and washed with 50 ml above mentioned solvent mixture [Ethyl acetate : Dichloromethane , 4:1], suck dry for 10 minutes
Wt. 90-92 gms wet cake
Again the obtained product is reslurried with 400 ml solvent mixture [Ethyl acetate : Dichloromethane , 4:1] at about 30-35°C for about 60 minutes, filtered and washed with 50 ml solvent mixture [Ethyl acetate : Dichloromethane , 4:1], dried in oven at 45-50°c for about 4 hours to give pure rosiglitazone.
Yield = 84-85 gms
LOD = Not more than 1.0 %
Chromatographic Purity = 98.5 -99.3 %
Any unknown individual impurity < 1.0%
Starting benzylidene impurity <0.15 %

## Claims

1. An improved process for the preparation of compounds of formula-I 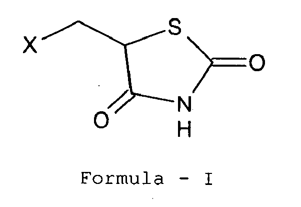 Wherein X is 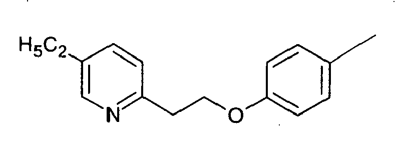 or 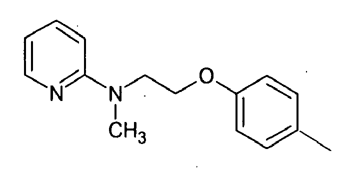 or 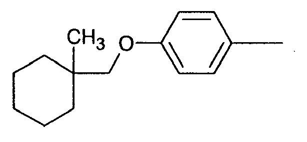 Wherein a compound of formula II 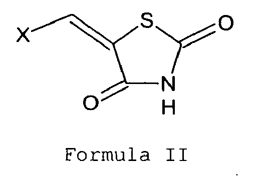 Wherein X is as defined above, is reduced in the steps comprising:
(a) dissolving compound of formula-II in DMF or formic acid and adding perchloric acid, reducing the arylidene compound to corresponding aryl methyl compound using a metal catalyst and hydrogen gas at elevated temperature and pressure,
(b) separating the catalyst, concentrating the filtrate, quenching in alcoholic water, followed by neutralizing and separating the compound of formula-I

2. A process for preparing a compound of formula I as claimed in Claim 1 wherein the preferred organic acid in step(a) is formic acid.

3. A process for preparing a compound of formula I as claimed in Claim 1, wherein the metal catalyst used in step(b) is selected from palladium, platinum , rhodium, ruthenium, iridium based metal catalysts.

4. A process for preparing a compound of formula I as claimed in Claim 1, wherein the metal catalyst in step (b) is 5-10 % palladium on carbon, more preferably 10 % palladium on carbon.

5. A process for preparing a compound of formula I as claimed in Claim 1, wherein the palladium [10 %] on carbon catalyst loading with respect to arylidene compound is 10 wt/wt to 80 % wt/wt, preferably 50 %.

6. A process for hydrogenation of thiazolidinedione precursor in perchloric acid and formic acid , with a supported metal catalyst such as palladium , platinum, rhodium, ruthenium, iridium at 50-100°C and pressure of 4 to 10 kg/ cm² preferably at 60-65°C and pressure of 5 Kg/cm².

7. A process for preparing a compound of formula I as claimed in Claim 1, wherein the alcohol in step-(b) is selected from C1- C3 alcohol, preferably isopropyl alcohol.

8. A process for purification of rosiglitazone comprising:
slurrying rosiglitazone in a mixture of [9:1] ethyl acetate and dichloromethane at 0-45°C , preferably at 20-35°C optionally in the presence of triethyl amine, for 15 minutes to 4 hours , separating the solids followed by drying.

9. A process of claim-[8] wherein the quantity of triethyl amine used is 1 to 10 vol /wt % of rosiglitazone, preferably 3to 5 vol /wt % of rosiglitazone.
